# EUROPEAN PATENT SPECIFICATION

(11) **EP 3 835 316 B1**
(45) Date of publication and mention of the grant of the patent: **21.10.2026**
(21) Application number: 19214551.4
(22) Date of filing: 09.12.2019
(51) Int. Cl.: C07K 14/705, A61K 38/00

(54) **MOLECULES FOR USE TO TREAT OR PREVENT CARDIAC DISEASES**
MOLEKÜLE ZUR VERWENDUNG ZUR BEHANDLUNG ODER VORBEUGUNG VON HERZKRANKHEITEN
MOLÉCULES À UTILISER POUR LE TRAITEMENT OU LA PRÉVENTION DE MALADIES CARDIAQUES

(43) Date of publication of application: 16.06.2021
(73) Proprietor: Universiteit Gent, 9000 Gent (BE); HOWEST, de Hogeschool West-Vlaanderen, 8500 Kortrijk (BE)
(72) Inventor: LEYBAERT, Luc, 9800 Bachte-Maria-Leerne (BE); LISSONI, Alessio, 9000 Gent (BE); HULPIAU, Paco, 8400 Oostende (BE); DE SMET, Maarten, 9185 Wachtebeke (BE)

(56) References cited:
- WO-A1-2015/017034
- WO-A2-2005/007090
- CN-A- 109 010 799
- US-A1- 2005 272 362
- US-A1- 2014 213 638
- DATABASE Geneseq [online] 7 March 2019 (2019-03-07), "Human RyR2 protein P1 domain, SEQ 3.", retrieved from EBI accession no. GSP:BFZ69891 Database accession no. BFZ69891
- DATABASE Geneseq [online] 9 February 2006 (2006-02-09), "5' end Turkey alphaRYR, SEQ ID 22.", XP002797938, retrieved from EBI accession no. GSP:AEE27596 Database accession no. AEE27596
- DATABASE Geneseq [online] 7 April 2005 (2005-04-07), "Human cardiovascular therapy target peptide CXA1.", retrieved from EBI accession no. GSP:ADW78032 Database accession no. ADW78032
- DATABASE Geneseq [online] 25 September 2014 (2014-09-25), "Human gap junction alpha-1 protein, SEQ ID 209.", XP002797939, retrieved from EBI accession no. GSP:BBK97830 Database accession no. BBK97830
- DATABASE Geneseq [online] 26 March 2015 (2015-03-26), "Mouse alpha connexin 43 (Cx43) fragment, SEQ ID 71.", retrieved from EBI accession no. GSP:BBU47117 Database accession no. BBU47117
- CORONEL R ET AL: "Electrophysiological changes in heart failure and their implications for arrhythmogenesis", BIOCHIMICA ET BIOPHYSICA ACTA. MOLECULAR BASIS OF DISEASE, AMSTERDAM, NL, vol. 1832, no. 12, 8 April 2013 (2013-04-08), pages 2432 - 2441, XP028768851, ISSN: 0925-4439, DOI: 10.1016/J.BBADIS.2013.04.002
- NAN WANG ET AL: "Connexin mimetic peptides inhibit Cx43 hemichannel opening triggered by voltage and intracellular Caelevation", BASIC RESEARCH IN CARDIOLOGY, STEINKOPFF-VERLAG, DA, vol. 107, no. 6, 21 October 2012 (2012-10-21), pages 1 - 17, XP035144358, ISSN: 1435-1803, DOI: 10.1007/S00395-012-0304-2

## Description

### Technical field of invention

The present invention relates to the treatment or prevention of certain cardiac diseases by targeting interactions between the ryanodine type-2 receptor (RyR2) protein and the connexin 43 (Cx43) protein. Indeed, the present invention relates to molecules -more specifically peptides- which target an FDFPDDN-containing interaction domain on the connexin 43 (Cx43) protein or which target an KNRRNPRL-containing interaction domain on the ryanodine type-2 receptor (RyR) protein, and which inhibit the opening of connexin 43 (Cx43) hemichannels present in cardiomyocytes. Hence, the latter molecules are useful to treat or prevent cardiac diseases in which the opening of Cx43 hemichannels is pathogenic.

### Background art

Cx43 is the most abundant connexin isotype in the heart. It is most prominently located at the level of the intercalated discs (IDs) of cardiomyocytes where it forms gap junctions (GJs) ¹. GJ channels are formed by the docking of two hemichannels (HCs) and given the high Cx43 turnover rate, this necessitates continuous supply of HCs through the perinexal area adjacent to the gap junctional plaques ¹⁻³. While GJs are normally open, HCs are closed under resting conditions; Cx43-based HCs open in response to various conditions including metabolic inhibition ^{4,5}, voltage steps to positive membrane potential ^{6,7}, alterations in the phosphorylation and redox status ⁸⁻¹¹, low extracellular Ca^{2+ 12,13}, or an increase in intracellular calcium concentration ([Ca²⁺]ᵢ) ^{7,14-16}. HC opening in response to [Ca²⁺]ᵢ elevation has only been documented by non-electrophysiological methods such as ATP release or dye uptake hemichannel assays, except for astrocytes where electrophysiological evidence is available ¹⁷. In cardiomyocytes, it has been demonstrated that Cx43 HC opening triggered by electrical stimulation to positive voltages is modulated by [Ca²⁺]ᵢ elevation, which lowers the voltage threshold for activation ⁷. However, the activation voltage threshold is high (+30 mV) and needs to be maintained for a sufficient period, leaving the question open whether such conditions can be realistically achieved during the cardiac action potential. Nothing is currently known concerning HC activation at negative diastolic membrane potentials. When open, Cx43 HCs behave as poorly selective channels, with a large pore characterized by a unitary conductance of ~220 pS, allowing transmembrane exchange of ions and small molecules that may affect electrochemical and metabolic cell homeostasis ^{7,14,18}. Cardiomyocytes express both RyR1 and RyR2 isoforms; RyR1 is found at mitochondria ⁴⁶⁻⁴⁸ or at IDs ⁴⁹. RyR2 is the most prevalent isoform in cardiac tissue ^{50,51}.

Connexinopathies involving Cx43 have been related to several cardiac diseases, such as sudden death, myocardial ischemia, ventricular arrhythmia, ventricular hypertrophy and recently to arrhythmogenic right ventricular cardiomyopathy (ARVC) ¹⁹⁻²³. GJs and disturbance of electrical conduction have been implicated in some of these diseases but recent evidence indicates that HCs should also be considered as potentially arrhythmogenic channels (see ref. 23). Consequently, new medicaments based on targeting connexin HCs and useful in treating the latter diseases are highly needed.

### Brief description of figures

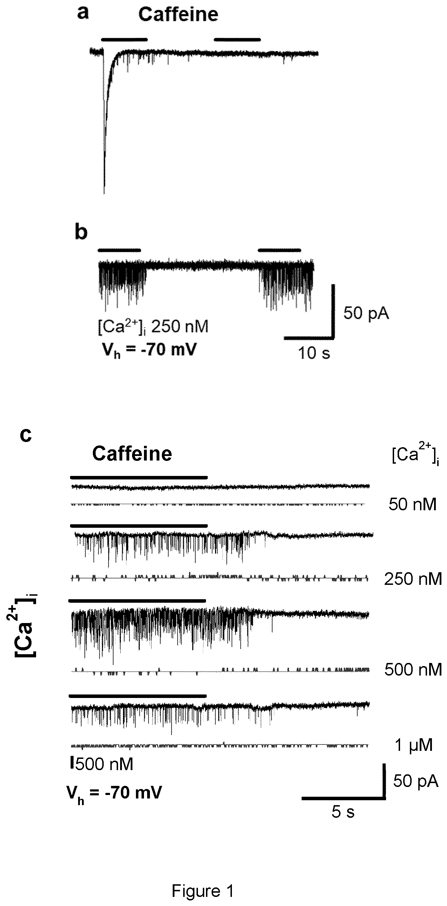

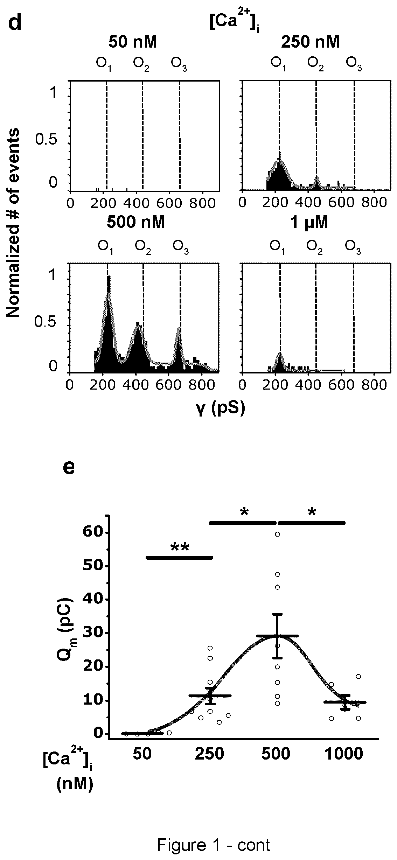

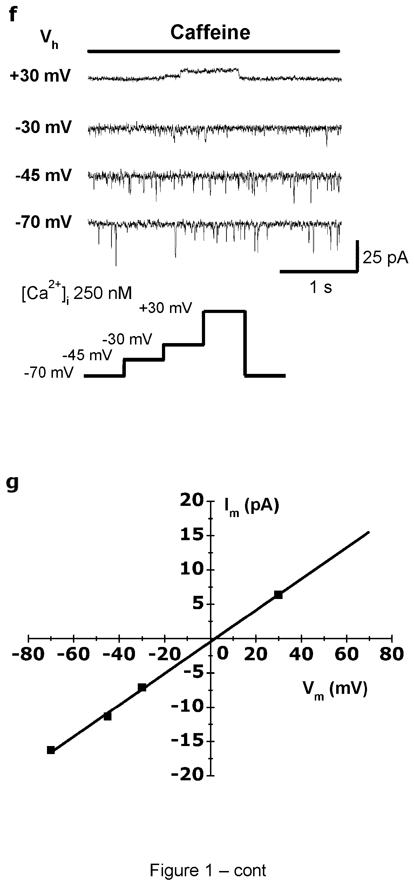

**Fig. 1****. Combined RyR activation/[Ca²⁺]ᵢ elevation is necessary to trigger unitary current activity.**
   **a.** Repeated caffeine exposure triggers unitary activity at the first but not at the second application.
   **b.** Under conditions of [Ca²⁺]ᵢ clamping to 250 nM (10 mM BAPTA), repeated RyR activation invariably provoked subsequent activation of unitary current activities. In between caffeine applications, 250 nM [Ca²⁺]ᵢ by itself was not sufficient to trigger unitary current activity.
   **c.** Representative current traces depicting unitary current activities obtained upon caffeine applications at different [Ca²⁺]ᵢ. RyR activation with [Ca²⁺]ᵢ set at 50 nM did not trigger unitary current activity, which only appeared upon elevating [Ca²⁺]ᵢ. The red traces represent associated [Ca²⁺]ᵢ recordings, illustrating stable [Ca²⁺]ᵢ clamping. [Ca²⁺]ᵢ shown are calculated values; see text for the corresponding measured values.
   **d.** Transition event histograms of each recording shown in (c). At 500 nM [Ca²⁺]ᵢ, increased HC opening activity can be clearly inferred from the increased frequency of double (O₂) and triple (O₃) stacked channel openings and corresponding conductance states, while further elevation to 1 µM reduced HC activity.
   **e.** Average data of unitary current integration over time representing membrane charge transfer (Qₘ) in the presence of various [Ca²⁺]ᵢ levels (50 nM n = 5, 250 nM n = 10, 500 nM n = 8, 1 µM n = 6; N = 5). RyR activation at 50 nM clamped [Ca²⁺]ᵢ does not activate HC opening while increasing [Ca²⁺]ᵢ to 250 and 500 nM gradually increases the opening response. 250 nM versus 50 nM [Ca²⁺]ᵢ *P* = 0.007 (unpaired Student's t-test). [Ca²⁺]ᵢ 250 nM versus 500 nM *P* = 0.0187; 1 µM versus 500 nM *P* = 0.023 (one-way ANOVA, Bonferroni posttest).
   **f.** Examples of current traces triggered by combined RyR activation and 250 nM [Ca²⁺]ᵢ at different membrane potentials. While negative potentials showed spiking unitary activities, positive potentials were characterized by much longer lasting outward currents associated with channel opening.
   **g.** Current-voltage plot based on the traces shown in (f), illustrating a slope conductance of 230 ± 2.8 pS and a reversal potential close to 0 mV, indicating involvement of a poorly selective channel (n = 10; N = 4).

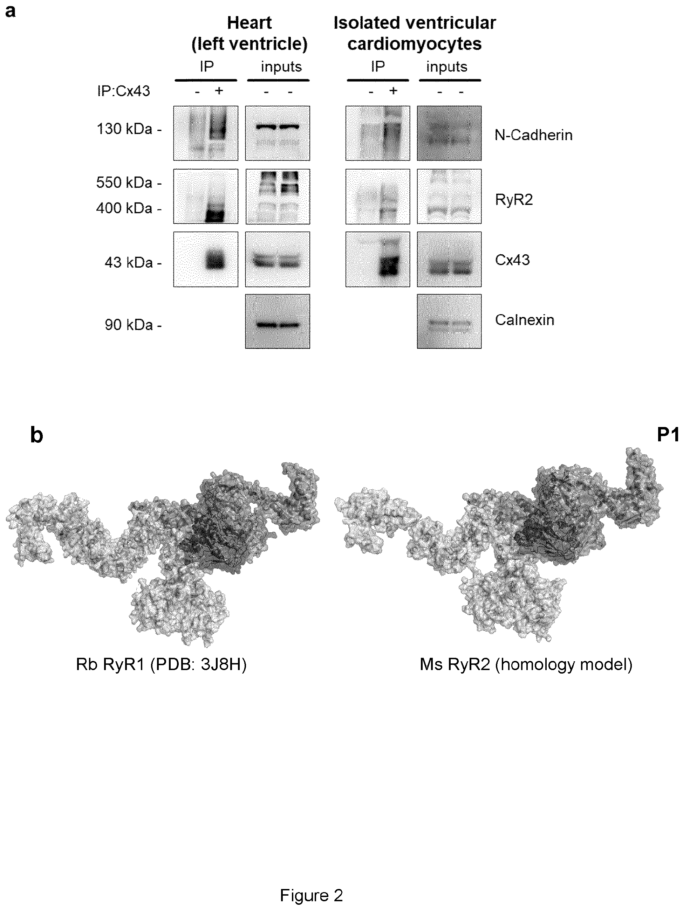

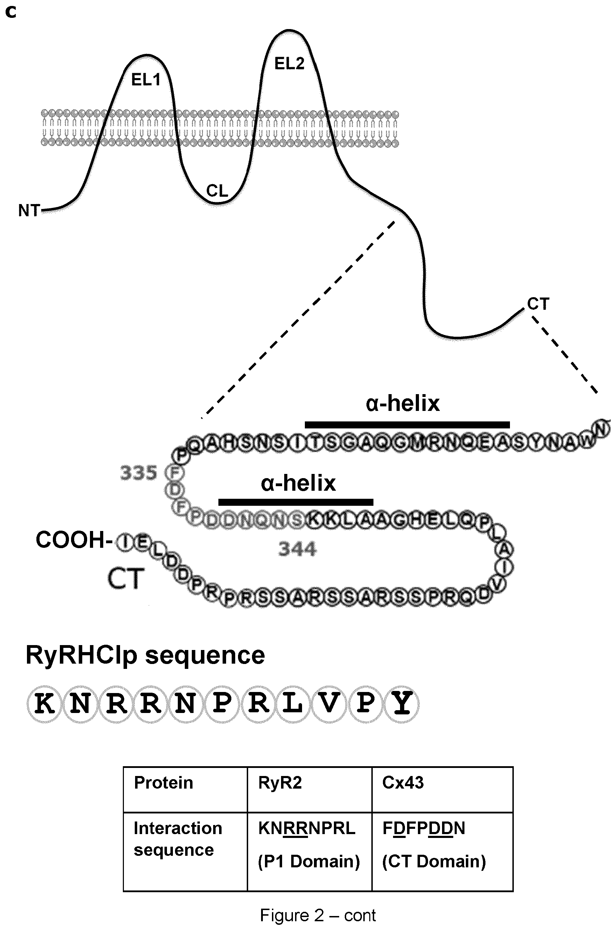

**Fig. 2****. Cx43 interacts with RyR2 in cardiomyocytes.**
   Co-IP and *in silico* studies of possible interaction sites between Cx43 and RyR2.
   **a.** Co-IP assays on whole heart (left column) and isolated cardiomyocytes lysates (right column). Cx43 co-precipitated with RyR2; N-cadherin was used as a positive control (N = 3). The second ~400 KDa RyR2 band has been reported to correspond to the C-terminal fraction of the channel ^{56,57}.
   **b.** Structure of rabbit RyR1 (PDB: 3J8H; left panel) and the homology model of mouse RyR2 cytoplasmic domain used for the docking study (1-2905; right panel).
   **c.** Schematic membrane topology of Cx43 with indication of the predicted binding region of the RyRHClp mimetic peptide on the C-terminal tail (CT) (sequence FDFPDDN from amino acid 335). The sequence partly overlaps with a second α-helical domain on the CT . The interaction sequences are reported in the table.

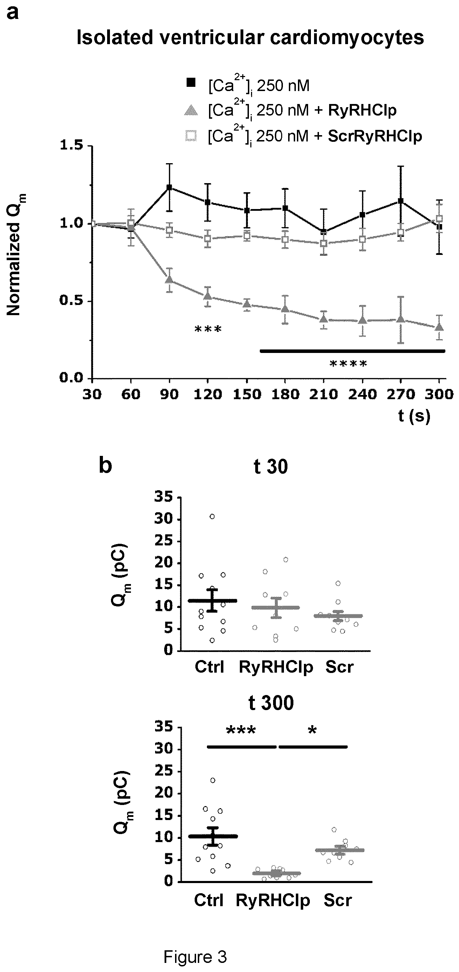

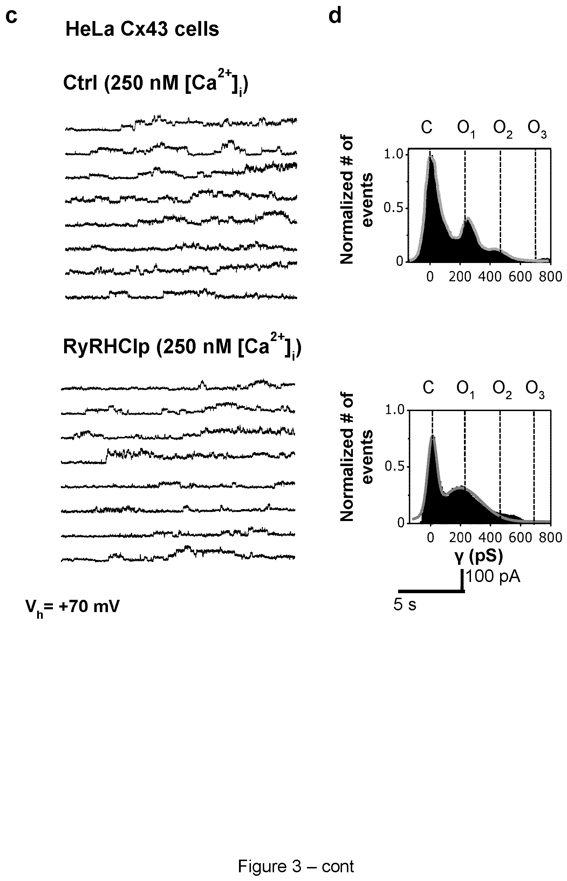

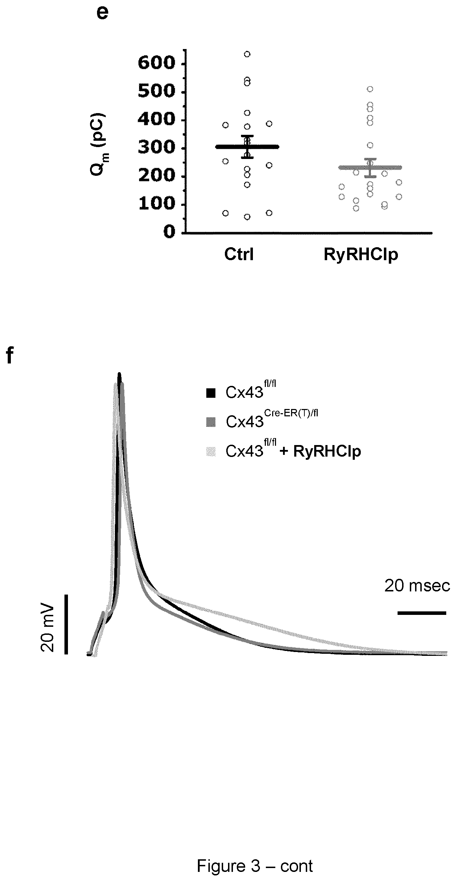

**Fig. 3****. RyRHClp inhibits HC activity triggered in cardiomyocytes and affects ventricular action potential, while it has no effect on electrically triggered HC currents in HeLa Cx43 cells.**
   **a.** Time course of RyRHClp inhibition of Cx43 HC unitary currents activated by RyR/[Ca²⁺]ᵢ elevation in cardiomyocytes. Qₘ values were normalized relative to the value at the 30 s timepoint for each condition. RyRHClp (250 µM) significantly decreased Cx43 HC opening activity , while scrambled RyRHClp (ScrRyRHClp; 250 µM) had no effect (Control 250 nM [Ca²⁺]ᵢ n = 11, RyRHClp n = 10, ScrRyRHClp n = 9; N = 5). 90 sec *P* = 0.028, 120 sec *P* = 0.0002, 150 sec *P* = 0.00003, 180 sec *P* = 0.00001, 210, 240, 270 and 300 sec *P* = <0.00001 versus t = 30 s (paired Student's t-test).
   **b.** Summary graph of non-normalized Qₘ data at 30 and 300 s. RyRHClp *P =* 0.0004, *P =* 0.042 versus control and ScrRyRHClp respectively (one-way ANOVA, Bonferroni posttest).
   **c.** Example current traces recorded in HeLa Cx43 cells in the absence or presence of RyRHClp (250 µM). Voltage steps to +70 mV were applied to induce Cx43 HC activity. **d.** All-point histograms of each set of recordings depicted in (c).
   **e.** Summary Qₘ data demonstrating no effect of RyRHClp (n = 20) on Cx43 HC (n = 18) activity in HeLa Cx43 cells (N = 4).
   **f.** Current clamp recordings of action potentials in cardiomyocytes, demonstrating the effect of Cx43 knockdown in Cx43^{Cre-ER(T)/fl} mice and of RyRHClp (250 µM) compared to measurements in Cx43^{fl/fl} control mice.

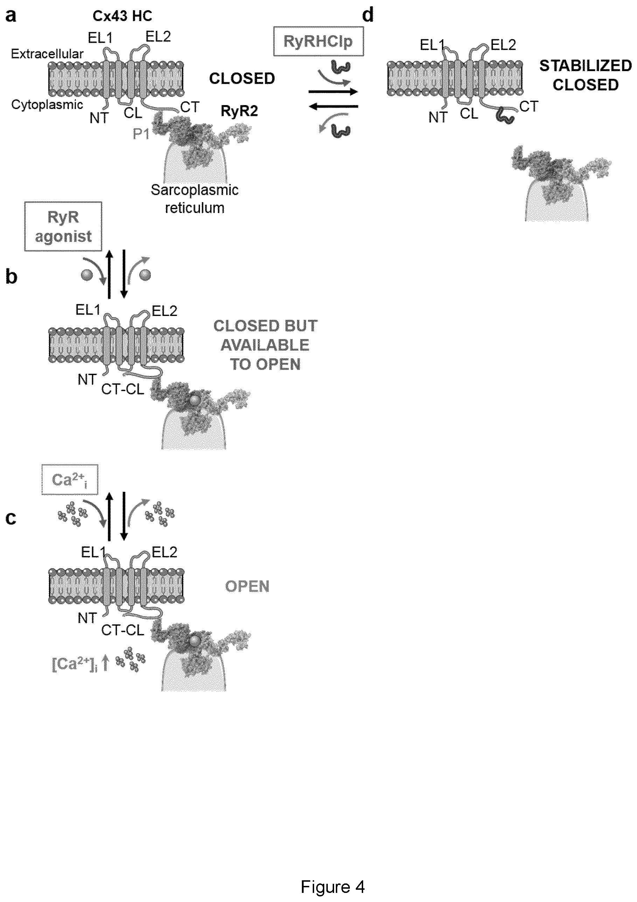

**Fig. 4****. Schematic model summarizing the findings of this study.**
   **a.** RyR2 and Cx43 interact and keep the channel in a closed state by preventing loop-tail interaction.
   **b.** Activation of RyR2 facilitates interaction of the CT with the cytoplasmic loop(CL) thereby bringing the channel in a closed but available to open state.
   **c.** [Ca²⁺]ᵢ elevation triggers the actual opening of Cx43 HCs.
   **d.** RyRHClp competes with RyR2 for binding to the Cx43 CT domain, thereby bringing the channel in a stabilized closed state.

### Description of invention

The present invention discloses that connexin 43 hemichannels -the most abundant connexin isotype in the heart-are intimately linked to ryanodine receptor type-2 -the most prevalent isotype in cardiac tissue-, allowing the connexin 43 hemichannels to open in response to RyR activation. The present invention shows that the sequence KNRRNPRL (SEQ ID N° 1) in the P1 domain of RyR interacts with the sequence FDFPDDN (SEQ ID N°2) of the CT domain of Cx43. Based on bioinformatics approaches, the present invention further discloses -as a non-limiting example- that a synthetized 11 amino acid RyR2 mimetic peptide having the sequence KNRRNPRLVPY inhibits opening of Cx43 hemichannels induced by calcium elevation/RyR activation (Fig 4). Hence, the present invention relates to molecules such as peptides, antibodies, small molecules and the like that interfere with the interaction between the sequence KNRRNPRL of the P1 domain of RyR2 and the sequence FDFPDDN of the CT domain of Cx43, thereby inhibiting the opening of Cx43 hemichannels induced by calcium elevation/RyR activation. In other words, the present invention relates to molecules as indicated above which target an FDFPDDN-containing interaction domain on the Cx43 protein or which target an KNRRNPRL-containing interaction domain on the RyR2 protein.

Hence, the present invention specifically relates to an isolated peptide which targets the FDFPDDN-containing interaction domain on the Cx43 protein and consists of the following amino acid sequences KNRRNPRLVPY (SEQ ID N°3). In other words, the present invention specifically relates to a biological oligomer consisting of the following chain of 11 amino acids which are linked together by peptide bonds ( symbolized by '-'): Lysine (Lys or K)- Asparagine (Asn or N)- Arginine (Arg or R)- Arginine (Arg or R)- Asparagine (Asn or N)- Proline (Pro or P)-Arginine (Arg or R)- Leucine (Leu or L)- Valine (Val or V)- Proline (Pro or P)-Tyrosine (Tyr or Y).

The peptide of the present invention can be obtained via chemical synthesis or by any other method known in the art.

As it is known that several cardiac diseases involve opening of Cx43 hemichannels, the present invention further relates to the latter isolated peptide for use as a medicament. The term 'medicament' relates to a substance used in therapy or treatment. In this regard, it should be clear that the sequence KNRRNPRLVPY -which is part of the P1 domain of the well-known RyR type-2 protein- is fully conserved in human, mice, rat, chicken and numerous other species.

The interacting FDFPDDN sequence on the Cx43 protein is conserved as well but to a lesser extent as compared to the KNRRNPRLVPY sequence.

Specifically, the present invention relates to the isolated peptide as described above for use to treat or prevent a cardiac disease.

More specifically, the present invention relates to an isolated peptide as described above wherein said cardiac disease is sudden death, myocardial ischemia, ventricular arrhythmia, ventricular hypertrophy or ARVC.

Moreover, the present invention relates to a pharmaceutical composition comprising a peptide as described above. The term 'a pharmaceutical composition' relates to a composition comprising the peptide as described above and which is further formulated to be compatible with its intended route of administration. Hence, suitable diluents, solvents, antioxidants, chelating agents, buffers, carriers, isotonic agents, binding agents, adjuvants, flavoring agents, propellants, detergents and the like which are described in detail in, for example, WO 03/004989, can be added to the peptide as described above.

The present invention also relates to a peptide or a pharmaceutical composition as described above for use in the treatment or prevention of a cardiac disease.

By the term 'treatment' is meant the medical management of a patient with the intent to cure, ameliorate, stabilize, or prevent a disease. It is further understood that appropriate doses of said compounds (which can also be denominated as drugs or pharmaceutical compositions) depends upon a number of factors within the knowledge of the ordinary skilled physician. The dose of these compounds will vary, for example, depending upon the identity, size, and condition of the patient being treated, upon the route of administration of said compounds (i.e. parenteral (intravenous, intradermal, subcutaneous), oral, transdermal, transmucosal or rectal) and upon the effect which the skilled physician desires the compound to have.

The present invention further relates to a peptide or a pharmaceutical compositionas described above wherein said cardiac disease is sudden death, myocardial ischemia, ventricular arrhythmia, ventricular hypertrophy or arrhytmogenic right ventricular cardiomyopathy.

The present invention further relates to the usage of the FDFPDDN-containing interaction domain on the Cx43 protein or the KNRRNPRL-containing interaction domain on the RyR protein to screen for molecules which interfere with the interaction between the sequence KNRRNPRL of the P1 domain of RyR and the sequence FDFDPDDN of the CT domain of Cx43 and which inhibit opening of Cx43 hemichannels induced by calcium elevation/ RyR activation. With the term 'molecules' are meant (stapled) peptides, antibodies or fragments thereof, molecules designed by nanobody-enabled reverse pharmacology as described by Pardon et al. (Angew. Chem. Ed. 2018, 57, 5292-5295), small molecules (i.e. a low molecular weight organic compound that regulates a biological process and with a size on the order of 1 nm) and the like.

The term 'screening assay' relates to any screening assay known in the art. In particular this involves interaction studies ('docking studies') between the FDFPDDN- and KNRRNPRL-containing domains making use of bioinformatics approaches. This is followed by validation of the found sequences in cell experiments and approaches to document and quantify the properties of interaction of the concerned sequences by e.g. surface plasmon resonance or microscale thermophoresis.

The following examples are provided to better illustrate the present invention and should not be considered as limiting the scope of the invention, which is defined by the claims.

### Examples

### Material and Methods:

### Cardiomyocyte isolation

Animal handling and procedures were approved by the Committee on ethical usage of animals of Ghent University and conformed to directive 2010/63/EU of the European Parliament.

Adult C57BL6 mice, between 10 - 16 weeks, were heparinized (5000 IU/kg IP) and sacrificed by cervical dislocation. Following thoracotomy, the heart was quickly excised and then transferred to a Langendorff apparatus and perfused at a constant flow (~3 mL/min) and temperature (37°C). The heart was digested using collagenase and protease and left ventricular isolated cardiomyocytes were collected. Ca²⁺-tolerant cells were used for the experiment for the next 6 hours. In case of Cx43 knockdown (KD) (Cx43^{CreER(T)/fl}) and respective control mice (Cre43^{fl/fl}) the isolation procedures were preceded by daily injection of tamoxifen to achieve global knockdown condition ²⁵. Cre43^{fl/fl} mice are available from The Jackson Laboratory and the Cx43^{CreER(T)/fl} transgenic mouse strain can be made by any method known in the art.

### Hela cell culture

HeLa Cells (up to passage 16) stably transfected with Cx43 (HeLa Cx43, ²⁶), were maintained in Dulbecco's modified Eagle's medium (Invitrogen, Gent, Belgium) and incubated at 37°C and 10 % CO₂.

### Immunocytochemistry and colocalization analysis

Colocalization analysis was performed on acute isolated mouse ventricular cardiomyocytes. The cells were stained with rabbit anti-Cx43 (Sigma-Aldrich, Cat no. C6219) and mouse anti-RyR C3-33 (Thermo Scientific, Cat no. MA3-916). Afterwards, confocal imaging was performed using a Leica XPS-8 with a water emersion objective 63X (numerical aperture 1.2). The Manders coefficient was used to quantify the degree of colocalization ²⁷.

### Proximity Ligation Assay

Adult C57BL6 mice were sacrificed as describe above and hearts were embedded in OCT medium (Klinipath) and then cut using a cryostat at 3 µm thickness. Proximity ligation was done on mouse ventricular slices according to manufacturer protocol (Sigma-Aldrich, Duolink kit DUO92101) to detect Cx43/RyR2 proximity. Rabbit anti-Cx43 (Sigma-Aldrich, Cat no. C6219) and mouse anti-RyR C3-33 (Thermo Scientific, Cat no. MA3-916) were used as primary antibodies. For the positive controls, we used mouse anti-Cx43 clone 4E6.2 (MerckMillipore, Cat no. MAB3067) and rabbit anti-Cx43.

### Western blot analysis

For Western blots, heart tissue samples collected during cardiomyocyte isolation were lysed and proteins were resolved in 4-12 % Bis-Tris gels (Invitrogen) and transferred to a nitrocellulose membrane (Amersham, Buckinghamshire, UK). Blots were probed with a rabbit anti-Cx43 antibody (SigmaAldrich) followed by alkaline phosphatase-conjugated goat anti-rabbit IgG antibody (Sigma-Aldrich). The blots were then developed with nitro blue tetrazolium/5-bromo-4-chloro-3-indolyl-phosphate reagent (Zymed, Invitrogen). Total protein stains by SYPRO Ruby (Invitrogen) prior to antibody staining was used as a loading control. Quantification was performed by using ImageJ.

### Immunoprecipitation assay

Co-immunoprecipitation (Co-IP) experiments were performed as described previously ²⁸. Lysates from both mouse whole hearts and isolated ventricular cardiomyocytes were prepared in lysis buffer. Determination of total protein was performed by the DC Protein Assay (BioRad, Hercules, CA), after which 1 mg of the supernatants were used for immunopurification. Proteins were resolved in 8% SDSpoly-acrylamide (SDS-PAGE) gel and transferred to a nitrocellulose membrane (BioRad, Hercules, CA). Blots were probed with primary antibodies against RyR2 (C3-33, Sigma-Aldrich), N-cadherin (H63, sc-7939; Santa Cruz Biotechnology, Heidelberg, Germany), Cx43 (AB0016) and Calnexin (AB0041, Sicgen), followed by appropriate horseradish peroxidase-conjugated IgG antibodies (BioRad). The proteins of interest were visualized by chemiluminescence using a VersaDoc system (Bio-Rad).

### Modeling and Docking

To predict putative interactions between the mouse RyR2 and the Cx43 hemichannel (Gja1) homology modeling and docking studies were performed. A homology model of mouse RyR2 was built with Modeller ²⁹. The model of mouse RyR2 cytoplasmic domain and carboxy-terminal domain of Cx43 were used for docking by ClusPro ³⁰ to predict binding of Cx43 to mouse RyR2. Homology and docking results were analyzed and figures were rendered using PyMOL.

### Peptide synthesis

Peptide synthesis was performed using solid phase peptide synthesis and reverse-phase HPLC purification to 95 %.

### Electrophysiological recording and intracellular calcium recording

Isolated ventricular cardiomyocytes and HeLa-Cx43 cells were studied under whole cell voltage-clamp to record membrane currents. Patch clamp experiments were conducted at room temperature (22 ± 1 °C). Fluo-4 (25 µM) was used to detect the [Ca²⁺]ᵢ transients during the current recording.

### Statistical analysis

Data are expressed as mean ± s.e.m with n giving the number of cells and N giving the number of independent experiments. Two groups were compared with a paired or unpaired Student's t-test and two-tail P value depending on the experiment design. Multiple groups were compared by one-way analysis of variance and a Bonferroni posttest, making use of OriginLab software (OriginLab Corporation, USA). Results were considered statistically significant when *P*< 0.05 (* for *P* < 0.05, ** for *P <* 0.01, *** for *P <* 0.001 and **** for *P* < 0.0001).

### Results:

### RyR agonists trigger unitary current activity with properties that resemble Cx43 HC opening

We used the RyR agonist caffeine (10 mM) to trigger currents recorded in whole cell mode from acutely isolated mouse ventricular cardiomyocytes voltage-clamped at a holding potential of -70 mV. Recordings were done in extracellular normal-Tyrode and intracellular KAsp based solutions (see Materials and methods). Caffeine (8 s) triggered a large inward current followed by a recovery phase that is known to be mediated by Na⁺/Ca²⁺ exchange (NCX) in response to the caffeine-triggered sarcoplasmic reticulum (SR) Ca²⁺ release ³². Superimposed on this macroscopic current were small spiking inward currents that appeared with a grossly similar amplitude. After subtraction of the NCX current these unitary events were more easily appreciated and all-events histogram analysis indicated a unitary conductance of ~220 pS, typical of Cx43 HCs ⁷. Cx43 HCs can be opened by modest [Ca²⁺]ᵢ elevation in voltage-clamped astrocytes held at -70 mV ¹⁷ and RyR-triggered [Ca²⁺]ᵢ elevation in response to caffeine may thus play a role in the presently observed unitary current activities. To investigate whether [Ca²⁺]ᵢ elevation by itself is sufficient to trigger unitary events, we performed experiments without caffeine and applied low extracellular Na⁺ (1 mM) to reverse the NCX working mode and let it pump in Ca²⁺ to obtain [Ca²⁺]ᵢ elevation ³³. Ryanodine (100 µM) was included in the pipette solution to avoid Ca²⁺-induced Ca²⁺ release ^{34,35}. In contrast to the caffeine experiments, no unitary current events were observed under those conditions, showing that [Ca²⁺]ᵢ elevation alone is not sufficient to elicit Cx43 HC opening.

### Combined RyR activation/[Ca²⁺]ᵢ elevation is necessary to trigger unitary current activity

Caffeine triggers two combined events, RyR activation and subsequent [Ca²⁺]ᵢ elevation, and we next aimed to separate both processes. To that purpose, we used a strongly Ca²⁺-buffered pipette solution containing 10 mM BAPTA to clamp [Ca²⁺]ᵢ to 50 (resting condition), 250, 500 and 1000 nM. These calculated [Ca²⁺]ᵢ values were verified by ratiometric fura-2 measurements, which resulted in actual Ca²⁺ concentrations of 49 ± 2, 283 ± 11, 505 ± 16 and 781 ± 25 nM (N = 3) respectively. For simplicity, we further refer to the calculated concentrations in the following text. Fig. 1a illustrates a current trace recorded with the normal pipette solution without added BAPTA (Vₘ = -70 mV), demonstrating that the first caffeine application triggered a large inward current with superimposed unitary current activity. However, the second caffeine application did not trigger any unitary current activity, indicating SR Ca²⁺ store depletion. By contrast, recordings with [Ca²⁺]ᵢ clamped to 250 nM showed quite different responses (Fig. 1b). First, caffeine application did not trigger any NCX current, which is considered to be a reliable reporter of [Ca²⁺]ᵢ in the subsarcolemmal compartment ³⁶⁻³⁸, indicating that [Ca²⁺]ᵢ is well clamped and stable. Second, unitary current activity was triggered not only by the first, but also by the second caffeine application, indicating that the [Ca²⁺]ᵢ imposed via the pipette is sufficient and SR Ca²⁺ release is not necessary. In line with this, the RyR inhibitors ryanodine and dantrolene ^{34,39,40} did not inhibit caffeine-induced Cx43 HC activation. Importantly, in between the two caffeine applications no unitary events occurred with 250 nM [Ca²⁺]ᵢ demonstrating that both [Ca²⁺]ᵢ elevation and caffeine activation of RyRs are necessary for triggering unitary activity. Fig. 1c illustrates experiments with simultaneous fluo-4 (25 µM in the pipette solution) recording of [Ca²⁺]ᵢ, demonstrating absence of unitary events when caffeine challenges were applied at resting [Ca²⁺]ᵢ of 50 nM ⁴¹ and appearance of unitary activities with caffeine applied at higher [Ca²⁺]ᵢ. As was already clear from the absence of NCX currents, the fluo-4 recordings were stable during caffeine applications confirming stable [Ca²⁺]ᵢ clamp. Histogram analysis showed a conductance peak at ~ 220 pS (225 ± 5 pS) in the 250 nM [Ca²⁺]ᵢ condition, while double and triple stacked openings superimposed on each other were present mostly at 500 nM [Ca²⁺]ᵢ were the Cx43 HC activity was higher (Fig. 1d). Average charge transfer (Qₘ) data of these experiments are shown in Fig. 1e, demonstrating a two-phased [Ca²⁺]ᵢ modulation of HC activity with [Ca²⁺]ᵢ below ~500 nM activating HC opening and concentrations above ~500 nM reducing HC activity. The activation has been linked to calmodulin-dependent signaling ¹⁴, while the limiting effect is, based on evidence obtained in non-muscle cells, the result of actomyosin contractility and disruption of Cx43 cytoplasmic loop/C-terminal tail interactions, which are necessary for HC opening ^{16,42} . Thus, Cx43 HCs can be opened by combined RyR/[Ca²⁺]ᵢ activation, but opening is counteracted when [Ca²⁺]ᵢ becomes too high.

We further explored the voltage dependence of the unitary current activities at 250 nM [Ca²⁺]ᵢ for different potentials and found a switch from fast spiking events at negative potential to long-lasting openings at positive potentials (Fig. 1f); the latter were identical to those described previously for pure voltage-triggered Cx43 HC openings ⁷. Interestingly, at positive voltage (+20 and +30 mV) caffeine was not necessary to obtain HC opening while it was indispensable to trigger HC opening at -70 mV (Fig. 1b). Graphical representation of these distinct opening responses demonstrated a linear I-V relationship characterized by a reversal potential of ~0 mV and a slope conductance of 230 ± 2.8 pS (n = 10) (Fig. 1g), which is in line with the histogram-based conductance analysis at -70 mV. The 230 pS conductance significantly differs from the 300-400 pS RyR or polycystin-2-like channel conductance that has previously been proposed to mediate sarcolemmal caffeine-triggered spiking inward currents ⁴³; it also differs from the Panx1 channel conductance reported to contribute to caffeine-triggered spiking current activity in atrial cells ⁴⁴. Our data show that RyR activation combined with [Ca²⁺]ᵢ elevation is necessary to activate Cx43 HC spiking inward current activity at -70 mV.

### Unitary current activity triggered by combined RyR/[Ca²⁺]ᵢ activation is strongly reduced in cardiomyocytes isolated from Cx43 knockdown animals

We determined whether the unitary event activity was dependent on Cx43 expression and applied the combined 250 nM [Ca²⁺]ᵢ/caffeine activation protocol on cardiomyocytes isolated from inducible global Cx43 knockdown animals. Charge transfer related to unitary current activity was on average 4-fold lower in Cx43^{Cre-ER(T)/fl} knockdown mice compared to Cx43 control mice (both strains received tamoxifen; see materials and methods) (not shown). Western blot analysis of Cx43 expression in ventricular heart lysates showed a ~10-fold decrease of Cx43 in Cx43^{Cre-ER(T)/fl} relative to Cx43^{fl/fl} animals. Taken together, these experiments demonstrate that the unitary event activity triggered by combined RyR activation/[Ca²⁺]ᵢ elevation is largely mediated by Cx43 HCs. As a control experiment, we tested whether the strongly decreased Cx43 expression in Cx43^{Cre-ER(T)/fl}-derived cardiomyocytes influenced the action potential properties in ventricular cardiomyocytes. We found a shorter APD₅₀ compared to control mice (Fig. 3f), shown previously to be linked to remodeling of K⁺ inward rectifier (I_{K1}), L-type Ca²⁺ and Nav1.5 channels ⁴⁵.

### Cx43 interacts with RyR2 at the level of the intercalated disc

Since co-activation of RyRs is necessary to trigger Cx43 HC opening by [Ca²⁺]ᵢ elevation, we verified whether an interaction between the two proteins is necessary. Cardiomyocytes express both RyR1 and RyR2 isoforms; RyR1 is found at mitochondria ⁴⁶⁻⁴⁸ or at IDs ⁴⁹. We here focused on RyR2 as the most prevalent isoform in cardiac tissue ^{50,51} and used an antibody strongly reactive for the RyR2 isoform. We first investigated *in situ* co-localization of Cx43 and RyR2, and then attempted to detect physical interactions between both proteins in ventricular cardiomyocytes. Double label immunofluorescence microscopy images of acutely isolated ventricular cardiomyocytes, indicates that ~24 % of the Cx43 signal co-localized with the RyR2 signal at the level of the ID. Tissue organization of RyR2 and Cx43 shows that the latter is located at the junctional area between adjacent cardiomyocytes ^{2,3,52}. Proximity ligation DuoLink studies targeting Cx43 and RyR2 showed positive signals between Cx43 and RyR2 at the IDs counterstained by pan-Cadherin immunostaining, suggesting apposition of the two proteins within less than 40 nm. As a positive control condition, we tested two different Cx43 antibodies recognizing distinct epitopes and found a typical ID-located distribution of the DuoLink signal. Negative control experiments performed by omitting one of the primary antibodies gave no DuoLink signal. To complement the immunochemistry-based approaches, we further assessed interactions between Cx43 and RyR2 by co-immunoprecipitation assays of protein from mouse adult heart lysates. These experiments demonstrated that RyR2 and N-cadherin co-precipitated with Cx43, both in whole heart lysates (Fig. 2a left) and lysates isolated from ventricular cardiomyocytes (Fig. 2a right) ²⁸. Overall these results strongly suggest a direct protein-protein interaction between Cx43 and RyR2 at ID-located areas in mouse cardiomyocytes.

### The RyR2 mimetic peptide RyRHClp inhibits Cx43 hemichannel opening induced by combined RyR/[Ca²⁺]ᵢ activation

The interaction between Cx43 and RyR2 was further investigated using ClusPro web server for proteinprotein docking analysis (see Material and Methods). For this study, a mouse RyR2 model based on the homotetrameric rabbit RyR1 3J8H (Fig. 2b ⁵³), and an NMR-based reconstruction of the C-terminal domain of Cx43 were used ⁵⁴. Following several simulation rounds, a common cluster region was identified between the P1 domain of RyR2 (sequence KNRRNPRL) and a corresponding interacting string on Cx43 located at the level of the second alpha helix of the CT domain (sequence FDFPDDN; Fig. 2c), involving several electrostatic interactions.

Based on these data, we synthetized an 11 amino acid RyR2 mimetic peptide, further called RyRHClp, that contained the predicted sequence with an additional 3 more conserved residues at its C-terminal end to increase binding selectivity (KNRRNPRLVPY), and subsequently tested its effect on Cx43 HC opening induced by [Ca²⁺]ᵢ elevation/RyR activation. RyRHClp (250 µM) was included in the 250 nM [Ca²⁺]ᵢ pipette solution, allowed to equilibrate for 2 minutes after breaking into the cell and followed by repetitive assessment of Cx43 HC activity every 30 sec. Fig. 3a-b shows the effect of the peptide compared to its scrambled version (ScrRyRHClp) and the control condition without peptide. RyRHClp significantly inhibited Cx43 HC opening within 2 min, while ScrRyRHClp as well as the control condition had no effect, as illustrated in the Qₘ plots.

We next verified whether RyRHClp would also affect HC activity in HeLa cells stably transfected with Cx43, which endogenously express RyR2 ⁵⁵, but lack the typical ventricular cardiomyocyte sarcolemmal organization. As a first experiment we tested whether caffeine (10 mM) could trigger spiking unitary HC current activity at negative potential, as observed in ventricular cardiomyocytes, but this was not the case. Subsequently, we elicited HC opening by voltage steps to positive Vₘ (+70 mV), and set [Ca²⁺]ᵢ to 250 nM to enhance electrically-triggered HC opening ⁷. Fig. 3c shows representative Cx43 HC current traces and corresponding all-point histograms demonstrating ~220 pS unitary event activity (Fig. 3d). Summary Qₘ data presented in Fig. 3e demonstrate that RyRHClp had no inhibitory effect on HC currents activated by voltage steps to positive potentials. Taken together, the spiking mode of HC activation at -70 mV is only observed in ventricular cardiomyocytes endowed with the necessary cytoarchitectural organization of RyR and Cx43 proteins, and RyRHClp only affects HC activity induced by combined RyR/[Ca²⁺]ᵢ activation, i.e. chemically activated HC opening, while not affecting electrically-triggered HC opening. In the last experiment we further tested whether RyRHClp would affect the action potential properties in cardiomyocytes and found the peptide to elongate the APD₉₀ (Fig. 3f).

### References

1. Delmar, M. & Liang, F.-X. Connexin43 and the regulation of intercalated disc function. Heart Rhythm 9, 835-8 (2012).
2. Rhett, J. M., Jourdan, J. & Gourdie, R. G. Connexin 43 connexon to gap junction transition is regulated by zonula occludens-1. Mol. Biol. Cell 22, 1516-28 (2011).
3. Rhett, J. M. & Gourdie, R. G. The perinexus: A new feature of Cx43 gap junction organization. Heart Rhythm 9, 619-623 (2012).
4. John, S. A., Kondo, R., Wang, S. Y., Goldhaber, J. I. & Weiss, J. N. Connexin-43 hemichannels opened by metabolic inhibition. J. Biol. Chem. 274, 236-40 (1999).
5. Kondo, R. P., Wang, S.-Y., John, S. A., Weiss, J. N. & Goldhaber, J. I. Metabolic Inhibition Activates a Non-selective Current Through Connexin Hemichannels in Isolated Ventricular Myocytes. J. Mol. Cell. Cardiol. 32, 1859-1872 (2000).
6. Contreras, J. E., Sáez, J. C., Bukauskas, F. F. & Bennett, M. V. L. Functioning of cx43 hemichannels demonstrated by single channel properties. Cell Commun. Adhes. 10, 245-9 (2003).
7. Wang, N., De Bock, M., Antoons, G. K., Gadicherla, A., Bol, M., Decrock, E., Howard, E. W., Sipido, K., Bukauskas, F. & Leybaert, L. Connexin mimetic peptides inhibit Cx43 hemichannel opening triggered by voltage and intracellular Ca 2+ elevation. Basic Res. Cardiol. 107, 1-17 (2012).
8. Retamal, M. A., Schalper, K. A., Shoji, K. F., Bennett, M. V. L. & Sáez, J. C. Opening of connexin 43 hemichannels is increased by lowering intracellular redox potential. Proc. Natl. Acad. Sci. U. S. A. 104, 8322-7 (2007).
9. Bao, X., Reuss, L. & Altenberg, G. A. Regulation of purified and reconstituted connexin 43 hemichannels by protein kinase C-mediated phosphorylation of Serine 368. J. Biol. Chem. 279, 20058-66 (2004).
10. Kim, D. Y., Kam, Y., Koo, S. K. & Joe, C. O. Gating connexin 43 channels reconstituted in lipid vesicles by mitogen-activated protein kinase phosphorylation. J. Biol. Chem. 274, 5581-7 (1999).
11. Johnstone, S. R., Billaud, M., Lohman, A. W., Taddeo, E. P. & Isakson, B. E. Posttranslational modifications in connexins and pannexins. J. Membr. Biol. 245, 319-32 (2012).
12. Stout, C. & Charles, A. Modulation of Intercellular Calcium Signaling in Astrocytes by Extracellular Calcium and Magnesium. Glia. 265-73. 43, (2003).
13. Stout, C. E., Costantin, J. L., Naus, C. C. G. & Charles, A. C. Intercellular Calcium Signaling in Astrocytes via ATP Release through Connexin Hemichannels. J Biol Chem. (12)10482-8. 277, (2002).
14. De Vuyst, E., Wang, N., Decrock, E., De Bock, M., Vinken, M., Van Moorhem, M., Lai, C., Culot, M., Rogiers, V., Cecchelli, R., Naus, C. C., Evans, W. H. & Leybaert, L. Ca2+ regulation of connexin 43 hemichannels in C6 glioma and glial cells. Cell Calcium 46, 176-187 (2009).
15. De Vuyst, E., Decrock, E., Cabooter, L., Dubyak, G. R., Naus, C. C., Evans, W. H. & Leybaert, L. Intracellular calcium changes trigger connexin 32 hemichannel opening. EMBO J. 25, 34-44 (2006).
16. Ponsaerts, R., De Vuyst, E., Retamal, M., D'hondt, C., Vermeire, D., Wang, N., De Smedt, H., Zimmermann, P., Himpens, B., Vereecke, J., Leybaert, L. & Bultynck, G. Intramolecular loop/tail interactions are essential for connexin 43-hemichannel activity. FASEB J. 24, 4378- 4395 (2010).
17. Meunier, C., Wang, N., Yi, C., Dallerac, G., Ezan, P., Koulakoff, A., Leybaert, L. & Giaume, C. Contribution of astroglial Cx43 hemichannels to the modulation of glutamatergic currents by D-serine in the mouse prefrontal cortex. J. Neurosci. 37, 2204-16 (2017).
18. Kang, J., Kang, N., Lovatt, D., Torres, A., Zhao, Z., Lin, J. & Nedergaard, M. Connexin 43 hemichannels are permeable to ATP. J. Neurosci. 28, 4702-11 (2008).
19. Basheer, W. A., Harris, B. S., Mentrup, H. L., Abreha, M., Thames, E. L., Lea, J. B., Swing, D. A., Copeland, N. G., Jenkins, N. A., Price, R. L. & Matesic, L. E. Cardiomyocyte-specific overexpression of the ubiquitin ligase Wwp1 contributes to reduction in Connexin 43 and arrhythmogenesis. J. Mol. Cell. Cardiol. 88, 1-13 (2015).
20. Leithe, E., Mesnil, M. & Aasen, T. The connexin 43 C-terminus: A tail of many tales. Biochim. Biophys. Acta - Biomembr. 1860, 48-64 (2018).
21. Garcia, I. E., Prado, P., Pupo, A., Jara, O., Rojas-Gómez, D., Mujica, P., Flores-Muñoz, C., González-Casanova, J., Soto-Riveros, C., Pinto, B. I., Retamal, M. A., González, C. & Martinez, A. D. Connexinopathies: a structural and functional glimpse. BMC Cell Biol. 17 Suppl 1 (2016).
22. Schulz, R., Görge, P. M., Görbe, A., Ferdinandy, P., Lampe, P. D. & Leybaert, L. Connexin 43 is an emerging therapeutic target in ischemia/reperfusion injury, cardioprotection and neuroprotection. Pharmacol. Ther. 153, 90-106 (2015).
23. Kim, J.-C., Pérez-Hernández Duran, M., Alvarado, F. J., Maurya, S. R., Montnach, J., Yin, Y., Zhang, M., Lin, X., Vasquez, C., Heguy, A., Liang, F.-X., Woo, S.-H., Morley, G. E., Rothenberg, E., Lundby, A., Valdivia, H. H., Cerrone, M. & Delmar, M. Disruption of Ca 2+ i Homeostasis and Cx43 Hemichannel Function in the Right Ventricle Precedes Overt Arrhythmogenic Cardiomyopathy in PKP2-Deficient Mice . Circulation (2019). doi:10.1161/circulationaha.119.039710
24. Wang, N., Vuyst, E. De, Ponsaerts, R., Boengler, K., Palacios-prado, N., Lai, C. P., Bock, M. De, Decrock, E., Bol, M., Vinken, M., Tavernier, J., Evans, W. H., Naus, C. C., Bukauskas, F. F., Sipido, R., Heusch, G., Schulz, R., Bultynck, G. & Leybaert, L. Selective inhibition of Cx43 hemichannels by Gap19 and its impact on myocardial ischemia/reperfusion injury. Basic Res Cardiol. 108, 1-26 (2013).
25. Boengler, K., Ruiz-Meana, M., Gent, S., Ungefug, E., Soetkamp, D., Miro-Casas, E., Cabestrero, A., Fernandez-Sanz, C., Semenzato, M., Lisa, F. Di, Rohrbach, S., Garcia-Dorado, D., Heusch, G. & Schulz, R. Mitochondrial connexin 43 impacts on respiratory complex I activity and mitochondrial oxygen consumption. J Cell Mol Med. 16, (8):1649-55 (2012).
26. Elfgang, C., Eckert, R., Lichtenberg-Fraté, H., Butterweck, A., Traub, O., Klein, R. A., Hülser, D. F. & Willecke, K. Specific permeability and selective formation of gap junction channels in connexin-transfected HeLa cells. J. Cell Biol. 129, 805-17 (1995).
27. Manders, E. M. M., Verbeek, F. J. & Aten, J. A. Measurement of co-localization of objects in dual-colour confocal images. J. Microsc. 169, 375-382 (1993).
28. Martins-Marques, T., Anjo, S. I., Pereira, P., Manadas, B. & Girão, H. Interacting Network of the Gap Junction (GJ) Protein Connexin43 (Cx43) is Modulated by Ischemia and Reperfusion in the Heart. Mol. Cell. Proteomics 14, 3040-55 (2015).
29. Webb, B. & Sali, A. Comparative Protein Structure Modeling Using MODELLER. Curr. Protoc. Bioinforma. 54, 5.6.1-5.6.37 (2016).
30. Kozakov, D., Beglov, D., Bohnuud, T., Mottarella, S. E., Xia, B., Hall, D. R. & Vajda, S. How good is automated protein docking? Proteins 81, 2159-66 (2013).
31. Higure, Y., Shimazaki, Y. & Nohmi, M. Can 4-chloro-m-cresol be substituted for caffeine as an activator of calcium oscillation in bullfrog sympathetic ganglion cells? Cell Calcium 39, 467-70 (2006).
32. Hilgemann, D. W. Regulation and deregulation of cardiac Na+-Ca2+ exchange in giant excised sarcolemmal membrane patches. Nature 344, 242-245 (1990).
33. Li, P. C., Yang, Y. C., Hwang, G. Y., Kao, L. Sen & Lin, C. Y. Inhibition of reverse-mode sodium-calcium exchanger activity and apoptosis by levosimendan in human cardiomyocyte progenitor cell-derived cardiomyocytes after anoxia and reoxygenation. PLoS One 9, 1-9 (2014).
34. Meissner, G. Ryanodine activation and inhibition of the Ca2+ release channel of sarcoplasmic reticulum. J. Biol. Chem. 261, 6300-6306 (1986).
35. Zimányi, I., Buck, E., Abramson, J. J., Mack, M. M. & Pessah, I. N. Ryanodine induces persistent inactivation of the Ca2+ release channel from skeletal muscle sarcoplasmic reticulum. Mol. Pharmacol. 42, (1992).
36. Varro, A., Negretti, N., Hester, S. B. & Eisner, D. A. An estimate of the calcium content of the sarcoplasmic reticulum in rat ventricular myocytes. Pflugers Arch. 423, 158-60 (1993).
37. Antoons, G., Mubagwa, K., Nevelsteen, I. & Sipido, K. R. Mechanisms underlying the frequency dependence of contraction and [Ca(2+)](i) transients in mouse ventricular myocytes. J. Physiol. 543, 889-98 (2002).
38. Weber, C. R., Piacentino, V., Ginsburg, K. S., Houser, S. R. & Bers, D. M. Na+-Ca2+ exchange current and submembrane [Ca2+] during the cardiac action potential. Circ. Res. 90, 182-189 (2002).
39. Thomas, N. L. & Williams, A. J. Pharmacology of ryanodine receptors and Ca 2+-induced Ca 2+ release. WIREs Membr Transp Signal 1, 383-397 (2012).
40. Hartmann, N., Pabel, S., Herting, J., Schatter, F., Renner, A., Gummert, J., Schotola, H., Danner, B. C., Maier, L. S., Frey, N., Hasenfuss, G., Fischer, T. H. & Sossalla, S. Antiarrhythmic effects of dantrolene in human diseased cardiomyocytes. Hear. Rhythm 14, 412-419 (2017).
41. Fiora, M., And, W.-G. & Isenberg, G. Total and free myoplasmic calcium during a contraction cycle: x-ray microanalysis in guinea-pig ventricular myocytes. J. Physiol. 435, 349-372 (1991).
42. Ponsaerts, R., Wang, N., Himpens, B., Leybaert, L. & Bultynck, G. The contractile system as a negative regulator of the connexin 43 hemichannel. Biol. Cell 104, 367-377 (2012).
43. Kondo, R. P., Weiss, J. N. & Goldhaber, J. I. Putative ryanodine receptors in the sarcolemma of ventricular myocytes. Pflugers Arch. Eur. J. Physiol. 440, 125-131 (2000).
44. Kienitz, M. C., Bender, K., Dermietzel, R., Pott, L. & Zoidl, G. Pannexin 1 constitutes the large conductance cation channel of cardiac myocytes. J. Biol. Chem. 286, 290-298 (2011).
45. Kontogeorgis, A., Li, X., Kang, E. Y., Feig, J. E., Ponzio, M., Kang, G., Kaba, R. A., Wit, A. L., Fisher, E. A., Morley, G. E., Peters, N. S., Coetzee, W. A. & Gutstein, D. E. Decreased connexin43 expression in the mouse heart potentiates pacing-induced remodeling of repolarizing currents. Am J Physiol Hear. Circ Physiol 295, 1905-1916 (2008).
46. Beutner, G., Sharma, V. K., Lin, L., Ryu, S.-Y., Dirksen, R. T. & Sheu, S.-S. Type 1 ryanodine receptor in cardiac mitochondria: Transducer of excitation-metabolism coupling. Biochim. Biophys. Acta - Biomembr. 1717, 1-10 (2005).
47. Jakob, R., Beutner, G., Sharma, V. K., Duan, Y., Gross, R. A., Hurst, S., Jhun, B. S., O-Uchi, J. & Sheu, S.-S. Molecular and functional identification of a mitochondrial ryanodine receptor in neurons. Neurosci. Lett. 575, 7-12 (2014).
48. O-Uchi, J., Jhun, B. S., Hurst, S., Bisetto, S., Gross, P., Chen, M., Kettlewell, S., Park, J., Oyamada, H., Smith, G. L., Murayama, T. & Sheu, S.-S. Overexpression of ryanodine receptor type 1 enhances mitochondrial fragmentation and Ca 2-induced ATP production in cardiac H9c2 myoblasts. Am J Physiol Hear. Circ Physiol 305, 1736-1751 (2013).
49. Jeyakumar, L. H., Gleaves, L. A., Ridley, B. D., Chang, P., Atkinson, J., Barnett, J. V. & Fleischer, S. The skeletal muscle ryanodine receptor isoform 1 is found at the intercalated discs in human and mouse hearts. J. Muscle Res. Cell Motil. 23, 285-292 (2002).
50. Sorrentino, V. & Volpe, P. Ryanodine receptors: how many, where and why? Trends Pharmacol. Sci. 14, 98-103 (1993).
51. Sutko, J. L. & Airey, J. A. Ryanodine receptor Ca2+ release channels: does diversity in form equal diversity in function? Physiol. Rev. 76, 1027-71 (1996).
52. Sato, P. Y., Coombs, W., Lin, X., Nekrasova, O., Green, K. J., Isom, L. L., Taffet, S. M. & Delmar, M. Interactions between ankyrin-G, Plakophilin-2, and Connexin43 at the cardiac intercalated disc. Circ. Res. 109, 193-201 (2011).
53. Yan, Z., Bai, X., Yan, C., Wu, J., Li, Z., Xie, T., Peng, W., Yin, C., Li, X., Scheres, S. H. W., Shi, Y. & Yan, N. Structure of the rabbit ryanodine receptor RyR1 at near-atomic resolution. Nature 517, 50-55 (2015).
54. Sorgen, P. L., Duffy, H. S., Sahoo, P., Coombs, W., Delmar, M. & Spray, D. C. Structural changes in the carboxyl terminus of the gap junction protein connexin43 indicates signaling between binding domains for c-Src and zonula occludens-1. J. Biol. Chem. 279, 54695-701 (2004).
55. Bennett, D. L., Cheek, T. R., Berridge, M. J., De Smedt, H., Parys, J. B., Missiaen, L. & Bootman, M. D. Expression and Function of Ryanodine Receptors in Nonexcitable Cells. J Biol Chem. (11)6356-62. 271, (1996).
56. Zissimopoulos, S., Seifan, S., Maxwell, C., Williams, A. J. & Lai, F. A. Disparities in the association of the ryanodine receptor and the FK506-binding proteins in mammalian heart. J. Cell Sci. 125, 1759-69 (2012).
57. Li, J., Imtiaz, M. S., Beard, N. A., Dulhunty, A. F., Thorne, R., vanHelden, D. F. & Laver, D. R. ß-Adrenergic Stimulation Increases RyR2 Activity via Intracellular Ca2+ and Mg2+ Regulation. PLoS One 8, e58334 (2013).

## Claims

1. An isolated peptide consisting of the following amino acid sequence: KNRRNPRLVPY.

2. An isolated peptide according to claim 1 for use as a medicament.

3. An isolated peptide according to claim 1 for use to treat or prevent a cardiac disease.

4. An isolated peptide according to claim 3 wherein said cardiac disease is sudden death, myocardial ischemia, ventricular arrhythmia, ventricular hypertrophy or arrhythmogenic right ventricular cardiomyopathy.

5. A pharmaceutical composition comprising a peptide according to claim 1

6. A pharmaceutical composition according to claim 5 for use in the treatment or prevention of a cardiac disease.

7. A pharmaceutical composition for use according to claim 6 wherein said cardiac disease is sudden death, myocardial ischemia, ventricular arrhythmia, ventricular hypertrophy or arrhytmogenic right ventricular cardiomyopathy.

8. The in vitro usage of the FDFPDDN-containing C-terminal tail (CT) domain on the Cx43 protein or the KNRRNPRL-containing interaction domain on the RyR protein to screen for molecules which interfere with the interaction between both said sequences and which inhibit opening of Cx43 hemichannels induced by calcium elevation/ RyR activation.

## Patentansprüche

1. Isoliertes Peptid, bestehend aus der folgenden Aminosäuresequenz: KNRRNPRLVPY.

2. Isoliertes Peptid nach Anspruch 1 zur Verwendung als Medikament.

3. Isoliertes Peptid nach Anspruch 1 zur Verwendung beim Behandeln oder Vorbeugen einer Herzerkrankung.

4. Isoliertes Peptid nach Anspruch 3, wobei es sich bei der Herzerkrankung um Sekundentod, myokardiale Ischämie, ventrikuläre Arrhythmie, ventrikuläre Hypertrophie oder arrhythmogene rechtsventrikuläre Kardiomyopathie handelt.

5. Pharmazeutische Zusammensetzung, umfassend ein Peptid nach Anspruch 1.

6. Pharmazeutische Zusammensetzung nach Anspruch 5 zur Verwendung bei der Behandlung oder Vorbeugung einer Herzerkrankung.

7. Pharmazeutische Zusammensetzung zur Verwendung nach Anspruch 6, wobei es sich bei der Herzerkrankung um Sekundentod, myokardiale Ischämie, ventrikuläre Arrhythmie, ventrikuläre Hypertrophie oder arrhythmogene rechtsventrikuläre Kardiomyopathie handelt.

8. In-vitro-Verwendung der FDFPDDN-enthaltenden C-terminalen Schwanz(CT= C-terminal tail)-Domäne auf dem Cx43-Protein oder der KNRRNPRL-enthaltenden Interaktionsdomäne auf dem RyR-Protein beim Screening auf Moleküle, die die Interaktion zwischen den beiden Sequenzen stören und die Öffnung von Cx43-Hemikanälen, die durch Calciumerhöhung/ RyR-Aktivierung induziert werden, hemmen.

## Revendications

1. Peptide isolé constitué de la séquence d'acides aminés suivante : KNRRNPRLVPY.

2. Peptide isolé selon la revendication 1 destiné à être utilisé comme médicament.

3. Peptide isolé selon la revendication 1 destiné à être utilisé pour traiter ou prévenir une maladie cardiaque.

4. Peptide isolé selon la revendication 3 dans lequel ladite maladie cardiaque est une mort subite, une ischémie myocardique, une arythmie ventriculaire, une hypertrophie ventriculaire ou une cardiomyopathie ventriculaire droite arythmogène.

5. Composition pharmaceutique comprenant un peptide selon la revendication 1.

6. Composition pharmaceutique selon la revendication 5 destinée à être utilisée dans le traitement ou la prévention d'une maladie cardiaque.

7. Composition pharmaceutique destinée à être utilisée selon la revendication 6, dans laquelle ladite maladie cardiaque est une mort subite, une ischémie myocardique, une arythmie ventriculaire, une hypertrophie ventriculaire ou une cardiomyopathie ventriculaire droite arythmogène.

8. Utilisation *in vitro* du domaine de queue C-terminal (CT) contenant FDFPDDN sur la protéine Cx43 ou du domaine d'interaction contenant KNRRNPRL sur la protéine RyR pour cribler des molécules qui interfèrent avec l'interaction entre lesdites deux séquences et qui inhibent l'ouverture des hémicanaux Cx43 induite par élévation du calcium/activation de RyR.
